# EUROPEAN PATENT APPLICATION

(11) **EP 1 905 781 A2**
(43) Date of publication of application: **02.04.2008**
(21) Application number: 06725825.1
(22) Date of filing: 17.03.2006
(51) Int. Cl.: C07K 14/705

(54) **NOVEL SOLUBLE EPCR PROTEIN OF NON-PROTEOLYTIC ORIGIN AND USE THEREOF**

(30) Priority: 17.03.2005 ES 200500623
(71) Applicant: Proyecto de Biomedicina Cima, S.L., 31180 Cizur Mayor (Navarra) (ES)
(72) Inventor: HERMIDA SANTOS José, Avda. Pio XII, 55 31008 Pamplona (Navarra) (ES); MONTES DÍAZ, Ramón, Avda. Pio XII, 55 31008 Pamplona (Navarra) (ES); MOLINA BUEY, Eva, Avda. Pio XII, 55 31008 Pamplona (Navarra) (ES); MARTÍNEZ ANSO, Eduardo, Avda. Pio XII, 55 31008 Pamplona (Navarra) (ES)
(74) Representative: Ungria Lopez, Javier
(86) International application number: PCT/ES2006/000136
(87) International publication number: WO 2006/097564

(57) **Abstract**

The invention relates to a novel soluble form of EPCR originating from an alternative splicing mechanism, and more specifically, a polypeptide comprising residues 201-256 of the sequence SEQ ID NO: 1 or a fragment of said sequence. The invention also relates to a recombinant or isolated EPCR protein comprising said polypeptide, an mRNA encoding a protein that comprises said polypeptide and to the use thereof in the detection of diseases. The invention further relates to an expression vector or a host cell comprising said polynucleotide, an expression system that comprises the expression vector and said cell, a specific isolated antibody for said polypeptide, and a method and a kit for the selective in vitro detection in a biological sample of an EPCR protein comprising sequence SEQ ID NO: 1.

SEQUENCE LISTING

<110>  Proyecto de Biomedicina CIMA S.L.

<120>  NOVEL SOLUBLE EPCR PROTEIN OF NON-PROTEOLYTIC ORIGIN AND USE
       THEREOF

<130>  FIMA04007

<160>  7

<170>  PatentIn version 3.1

<210>  SEQ ID NO:1

<211>  256

<212>  PRT

<213>  Homo sapiens

<220>

<221>  SIGNAL

<222>  (1)..(17)

<223>  signal peptide

<220>

<221>  MISC_FEATURE

<222>  (18)..(200)

<223>  EPCR common extracellular domain

<220>

<221>  DOMAIN

<222>  (201)..(256)

<223>  Differential domain of the novel form of soluble EPCR

<400>  1

Met Leu Thr Thr Leu Leu Pro Ile Leu Leu Leu Ser Gly Trp Ala Phe
1               5                   10                  15

Cys Ser Gln Asp Ala Ser Asp Gly Leu Gln Arg Leu His Met Leu Gln
            20                  25                  30

Ile Ser Tyr Phe Arg Asp Pro Tyr His Val Trp Tyr Gln Gly Asn Ala
        35                  40                  45

Ser Leu Gly Gly His Leu Thr His Val Leu Glu Gly Pro Asp Thr Asn
    50                  55                  60

Thr Thr Ile Ile Gln Leu Gln Pro Leu Gln Glu Pro Glu Ser Trp Ala
65                  70                  75                  80

Arg Thr Gln Ser Gly Leu Gln Ser Tyr Leu Leu Gln Phe His Gly Leu
                85                  90                  95

Val Arg Leu Val His Gln Glu Arg Thr Leu Ala Phe Pro Leu Thr Ile
            100                 105                 110

Arg Cys Phe Leu Gly Cys Glu Leu Pro Pro Glu Gly Ser Arg Ala His
        115                 120                 125

Val Phe Phe Glu Val Ala Val Asn Gly Ser Ser Phe Val Ser Phe Arg
    130                 135                 140

Pro Glu Arg Ala Leu Trp Gln Ala Asp Thr Gln Val Thr Ser Gly Val
145                 150                 155                 160

Val Thr Phe Thr Leu Gln Gln Leu Asn Ala Tyr Asn Arg Thr Arg Tyr
                165                 170                 175

Glu Leu Arg Glu Phe Leu Glu Asp Thr Cys Val Gln Tyr Val Gln Lys
            180                 185                 190

His Ile Ser Ala Glu Asn Thr Lys Asp Asn Gly Val Gly Ala Gly Ser
        195                 200                 205

Leu Trp Pro Ile Leu Gln Arg Gln Thr Glu Ser Pro Glu Ala Phe Lys
    210                 215                 220

Arg Tyr Asn Gln Ile Asn Lys Ser Ser Thr Ile Lys Ile Gln His Ser
225                 230                 235                 240

Ile Leu Pro Gly Val Ser Asp Leu Gly Trp Asp Ala Asp Ile Ile Gly
                245                 250                 255

<210> SEQ ID NO:2

<211> 771

<212> DNA

<213> Homo sapiens

<400> 2
atgttgacaa cattgctgcc gatactgctg ctgtctggct gggccttttg tagccaagac 60

gcctcagatg gcctccaaag acttcatatg ctccagatct cctacttccg cgacccctat 120

cacgtgtggt accagggcaa cgcgtcgctg gggggacacc taacgcacgt gctggaaggc 180

ccagacacca acaccacgat cattcagctg cagcccttgc aggagcccga gagctgggcg 240

cgcacgcaga gtggcctgca gtcctacctg ctccagttcc acggcctcgt gcgcctggtg 300

caccaggagc ggaccttggc ctttcctctg accatccgct gcttcctggg ctgtgagctg 360

cctcccgagg gctctagagc ccatgtcttc ttcgaagtgg ctgtgaatgg gagctccttt 420

gtgagtttcc ggccggagag agccttgtgg caggcagaca cccaggtcac ctccggagtg 480

gtcaccttca ccctgcagca gctcaatgcc tacaaccgca ctcggtatga actgcgggaa 540

ttcctggagg acacctgtgt gcagtatgtg cagaaacata tttccgcgga aaacacgaaa 600

gataatggag ttggggcagg aagcctatgg cccatcctcc aaagacagac agaatcacct 660

gaggcgttca aaagatataa ccaaataaac aagtcatcca caatcaaaat acaacattca 720

atacttccag gtgtgtcaga cttgggatgg gacgctgata taatagggta g 771

<210> SEQ ID NO: 3

<211> 36

<212> DNA

<213> Artificial

<400> 3

agcttggcat atcgattagc caagacgcct cagatg 36

<210> SEQ ID NO: 4

<211> 36

<212> DNA

<213> Artificial

<400> 4

agctatcgta gcggccgcct accctattat atcagc 36

<210> SEQ ID NO: 5

<211> 26

<212> DNA

<213> Artificial

<400> 5

cagcgaacgc ttggccctgg taccac 26

<210> SEQ ID NO: 6

<211> 25

<212> DNA

<213> Artificial

<400> 6

gctcaatgcc taccaacgca ctcgg 25

<210> SEQ ID NO: 7

<211> 831

<212> DNA

<213> Artificial

<400> 7
aacccaggtc cggagcctca acttcaggat gttgacaaca ttgctgccga tactgctgct 60

gtctggctgg gccttttgta gccaagacgc ctcagatggc ctccaaagac ttcatatgct 120

ccagatctcc tacttccgcg acccctatca cgtgtggtac cagggcaacg cgtcgctggg 180

gggacaccta acgcacgtgc tggaaggccc agacaccaac accacgatca ttcagctgca 240

gcccttgcag gagcccgaga gctgggcgcg cacgcagagt ggcctgcagt cctacctgct 300

ccagttccac ggcctcgtgc gcctggtgca ccaggagcgg accttggcct ttcctctgac 360

catccgctgc ttcctgggct gtgagctgcc tcccgagggc tctagagccc atgtcttctt 420

cgaagtggct gtgaatggga gctcctttgt gagtttccgg ccggagagag ccttgtggca 480

ggcagacacc caggtcacct ccggagtggt caccttcacc ctgcagcagc tcaatgccta 540

caaccgcact cggtatgaac tgcgggaatt cctggaggac acctgtgtgc agtatgtgca 600

gaaacatatt tccgcggaaa acacgaaaga taatggagtt ggggcaggaa gcctatggcc 660

catcctccaa agacagacag aatcacctga ggcgttcaaa agatataacc aaataaacaa 720

gtcatccaca atcaaaatac aacattcaat acttccaggt gtgtcagact tgggatggga 780

cgctgatata atagggtaga aagaagtaac acgaagaagt ggtggaaatg t 831

## Description

### BACKGROUND OF THE INVENTION

The last protein of the protein C system (PC) described is endothelial protein C/activated protein C receptor (EPCR) (1, 2). EPCR is expressed on the membrane of endothelial cells and binds PC and activated protein C (APC) with high affinity (Kd - 30 nM). Its physiological mission is to concentrate PC on the endothelial surface and present it to the thrombin-thombomodulin complex, thus favouring effective activation of PC (3). In studies performed on non-human primates, it has been demonstrated that blocking EPCR with a monoclonal antibody reduces the physiological production of APC by 90% and recent studies suggest that genetic defects of EPCR may predispose to suffering from venous and arterial thrombosis (4-7). These facts demonstrate the important role that EPCR may play in coagulation regulation and in thrombosis. Furthermore, EPCR seems to be one of the molecules responsible for the powerful anti-inflammatory effect of PC.

The interaction of PC/APC with EPCR may play a crucial role in modulating the coagulopathic and inflammatory response developed during infection by gram negative germs (8). Recently, it has been demonstrated that APC has an anti-inflammatory and antiapoptotic effect on endothelial cells, and that presence of EPCR is necessary for this effect to be evident (9,10).

EPCR is a glycoprotein of approximately 46 kDa. The mature protein is comprised of 221 amino acids after elimination of the signal peptide, which consists of 17 residues (11). It is coded by a gene localised in the long arm of chromosome 20 (20q11.2), formed by four exons interrupted by three introns (12). Exon I encodes a 5' untranslated region and the signal peptide, exon IV codes the transmembrane domain, the intracytoplasmatic tail of three residues and the 3' untranslated region. Exons II and III encode most of the extracellular region of EPCR, which has homology with domains α1 and α2 of proteins belonging to the CD1 superfamily of class I the major histocompatibility complex. Recently, the three-dimensional structure which shows how EPCR has a platform formed by folded β sheets, whereon two with an α helix conformation, which form a pocket wherein a phospholipid is housed which stabilises the molecule, has managed to be crystallised and analysed (13). Likewise, by means of alanine scanning the main residues involved in the bond with PC/APC have been identified, to which it binds via its GLA domain in the presence of calcium and magnesium ions (14).

But, in addition to the EPCR anchored on the endothelial cell membrane, there is a plasma-soluble form (sEPCR) which, at least partially, comes from the digestion of EPCR by metalloproteinases induced by stimuli such as thrombin (15, 16).

Article (15) J. Clin. Invest. Vol. 100, no.2 July 1997, 411-418, relates to a soluble form of EPCR found in plasma. It states that the EPCR in plasma seems to be a unique species, and sequencing of the amino terminal fragment of the purified protein gave a unique sequence of plasma EPCR, which coincided with the amino terminal sequence of recombinant sEPCR. It states that as in the case of other receptors, the origin of said soluble EPCR may be proteolytic rupture, or an alternate cut-splice mechanism, and specifically the genomic structure of bovine EPCR contains an alternate cut site which encodes a protein wherein the transmembrane domain is replaced by a sequence encoded by the intron which is localised after exon III (17). This document indicates that more studies will be needed to prove the possibility of a form of EPCR derived from alternate cutting.

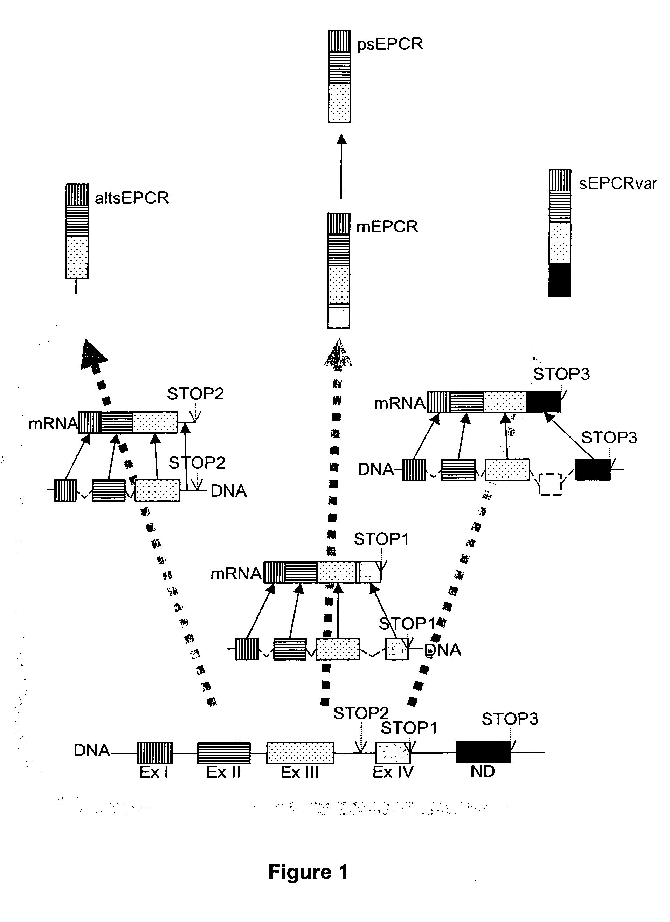

Furthermore, application WO-9900673 relates to the isolation and characterisation of sEPCR in human plasma and it states that the sEPCR may come from proteolysis, wherein an extracellular domain of EPCR is released and leaves the rest of the protein bound to the membrane, or from an alternate cut-splice mechanism of the mRNA which gives rise to the sequence of altsEPCR. Figure 1 of the present application shows a diagram wherein the difference between soluble EPCR of proteolytic origin (psEPCR), the EPCR disclosed in application WO-9900673 (altsEPCR) obtained by alternate cut-splice and the new EPCR object of the present invention (sEPCRvar) is observed.

The present application therefore indicates that at least human EPCR, can undergo an alternate cut-splice process, giving rise to a soluble truncated EPCR, which includes a unique insert present only in soluble EPCR derived from said alternate cut-splice mechanism (sEPCRvar). Said sEPCRvar may serve as a disease process marker in large vessels and in cancer.

When APC binds to sEPCR, it loses its anticoagulant properties but maintains its enzymatic capacity, for this reason it is thought that sEPCR alters APC specificity, possibly directing it towards a substrate, unknown at present, which may be connected to the anti-inflammatory properties attributed to APC (18). sEPCR competes with the EPCR present on the membrane for protein C and in this way inhibits APC generation. It has also been described that sEPCR is capable of interacting with activated neutrophils through proteinase-3 (PR3) (19), a proteinase present in neutrophil granules and Mac-1 (CD11b/CD18), an integrin of inducible expression, present on the surface of neutrophils and monocytes). PR3 is probably involved in tissue destruction mechanisms acting on membrane-bound TNF-alpha and IL-1beta precursors. Mac-1 intervenes in cell signalling and intercellular adhesion phenomena, interacting with adhesion molecules such as endothelial ICAM-1, reason for which it plays a role in recruiting neutrophils during inflammatory processes. Presumably, the binding of sEPCR to PR3 and Mac-1 would reduce their activity.

Starting from human genomic DNA (cDNA) the inventors have been able to clone and express a new protein. The analysis and comparison of its sequence with other known sequences, as well as its functional characterisation, have demonstrated that said protein is an unknown form of soluble EPCR protein generated by alternative splicing processes. On the other hand, said protein has as a differential feature a polypeptidic region encoded by the region originally considered as the EPCR gene 3' untranslated region, which isn't present in the EPCR forms presently known.

### DESCRIPTION OF THE INVENTION

The present invention firstly relates to a novel soluble form of EPCR, which we call soluble EPCR variant (sEPCRvar), more specifically to a polypeptide derived from an endothelial protein C receptor which comprises residues 201-256 of the sequence SEQ ID NO:1, or a fragment of said sequence of residues. In a particular embodiment, said fragment has at least 9 amino acids. According to particular embodiments of the invention, said fragment is selected from a fragment which comprises residues 213-227, 229-242, 212-226, 227-241, 242-256 of SEQ ID NO:1.

In a particular embodiment, said fragment has immunogenic properties and it is particularly useful in procedures for the production of specific antibodies.

The present invention further relates to an isolated or recombinant protein which comprises a polypeptide formed from residues 201-256 of the sequence SEQ ID NO:1. Said protein is an EPCR protein.

According to a preferred embodiment of the invention, said isolated or recombinant EPCR protein comprises the sequence SEQ ID NO:1.

An additional object of the present invention is a recombinant protein which comprises a polypeptide formed from residues 201-256 of the sequence SEQ ID NO:1, or a fragment of said sequence of residues.

An additional object of the present invention is a polynucleotide which comprises a sequence which encodes a polypeptide formed by residues 201-256 of sequence SEQ ID NO:1, or a fragment of said sequence of residues. In a particular embodiment, said polynucleotide comes from a cDNA sequence corresponding to SEQ ID NO:2. This cDNA relates to DNA retrotranscribed from mRNA.

In a particular embodiment of the invention, the polynucleotide of the invention may be contained in a DNA construct. Thus, an additional object of the invention is a DNA construct which encodes a protein or polypeptide which comprises residues 201-256 of sequence SEQ ID NO:1, or a fragment of said sequence of residues. Preferably, said fragment has at least 9 amino acids. In a particular embodiment of said construct, it comprises a polynucleotide which comes from a cDNA sequence corresponding to SEQ ID NO:2. Said DNA construct may incorporate a control sequence operatively bound to said protein or polypeptide. Concerning nucleic acids or polynucleotides, "operatively bound" means that one nucleotide region is put in functional relationship with another nucleotide sequence. "Control sequences" are expression signals, specifically recognised by a certain host cell, which regulate functions such as, for example, transcription and translation of a certain coding sequence. Promoters, expression enhancers, transcription terminators, ribosome binding sites, etc. are control sequences. The splicing of the desired sequences to form the DNA construct can be performed by cutting and splicing in appropriate restriction sites. If these binding sites do not exist, it is possible to generate them by conventional genetic engineering methods using synthetic oligonucleotides as adapters or spacers.

The polynucleotide or DNA construct of the invention may be obtained by conventional genetic engineering methods, frequently set down in laboratory manuals.

The polynucleotide or construct of the invention may be inserted in a suitable expression vector. Thus, an additional object of the present invention is an expression vector that comprises said polynucleotide or DNA construct. The choice of expression vector in the different embodiments of the invention will depend on the host cell wherein said expression vector is going to be inserted. By way of example, the vector wherein the polynucleotide or DNA construct of the invention is inserted may be a plasmid or a virus, which, once introduced in the host cell, may or may not integrate in the cell genome. This expression vector can also be obtained by conventional methods.

An additional object of the invention is a transformed host cell which comprises a polynucleotide or DNA construct which encodes a polypeptide which comprises residues 201-256 of sequence SEQ ID NO: 1, or a fragment of said sequence of residues with at least 9 amino acids. According to the invention, the transformed host cell may be a prokaryote cell, e.g. *Escherichia coli,* or a eukaryote, e.g. a yeast (among others *Pichia Pastoris* and *Saccharomyces cerevisiae),* an insect cell or a mammal cell line.

In a particular embodiment of the invention, a polynucleotide of the invention with SEQ ID NO:2 is introduced in *Pichia Pastoris* to produce a recombinant sEPCR protein of SEQ ID NO:1, corresponding to the novel form of sEPCR generated by alternate cutting and splicing disclosed in the present invention (sEPCRvar). The methods used to generate the DNA constructs, the expression vectors and transformed host cells necessary to produce the recombinant protein are described in detail later on, including the process for their purification.

In a particular embodiment of the invention the expression vector that includes the polynucleotide or DNA construct of the invention, is designed for its use in compositions and processes of in vivo gene transfer or therapy. In a more particular embodiment, this expression vector is a viral vector. Suitable viral vectors for this purpose include adenoviral, adenoassociated, retroviral, lentiviral, alphaviral, herpesviral, coronavirus derivative vectors, etc.

An additional object of the present invention is an expression system which comprises an expression vector which comprises a polynucleotide which encodes a polypeptide which comprises residues 201-256 of sequence SEQ ID NO:1, or a fragment of said sequence of residues, and a host cell transformed with said polynucleotide or said construct. Preferably, said fragment comprises at least 9 amino acids.

An additional object of the invention is a method to produce a protein or polypeptide which comprises residues 201-256 of SEQ ID NO: 1, or a fragment of said sequence of residues, preferably with at least 9 amino acids, said method characterised in that it comprises the culture of a host cell which comprises a polynucleotide or DNA construct of the invention in conditions which allow the expression of said protein, polypeptide or fragment. The conditions to optimise the culture will depend on the host cell used. If desired, this method may also include some steps to isolate and purify the protein or polypeptide expressed.

Alternatively, the protein or polypeptide of the invention can be obtained by other conventional methods, e.g. by chemical synthesis on solid phase techniques; purifying by high performance liquid chromatography (HPLC); and, if desired, they can also be analysed by conventional techniques, e.g. by sequencing and mass spectrometry, amino acid analysis, nuclear magnetic resonance, etc.

An additional object of the present invention is a mRNA which encodes a protein which comprises a sequence SEQ ID NO: 1, or a fragment of said sequence of residues.

An additional object of the present invention is a specific isolated antibody for a polypeptide which comprises residues 201-256 of SEQ ID NO: 1, or a fragment of said sequence of residues with immunogenic properties. In a particular embodiment, said fragment possesses at least 9 amino acids.

In a particular embodiment, said antibody is specific for a polypeptide or fragment whose sequence is selected from residues 201-256, 213-227, 229-242, 212-226, 227-241 and 242-256, all referred to SEQ ID NO:1.

In another particular embodiment, said antibody is specific for SEQ ID NO: 1 and does not recognise region 1-200 when it is not bound to region 201-256.

According to a preferred embodiment, said antibody is a monoclonal antibody.

An additional object of the present invention is a method for the selective in vitro detection in a biological sample, of an EPCR protein which comprises residues 201-256 of sequence SEQ ID NO:1, e.g. the new EPCR protein of SEQ ID NO:1, or a fragment of said sequence of residues, which comprises:
- obtaining a biological sample,
- analysing the quantity of EPCR protein which comprises residues 201-256 of sequence SEQ ID NO:1, or a fragment of said sequence of residues.

Said biological sample may be any sample that includes biological material. In a preferred embodiment, said biological sample is a urine, plasma, serum, tissue or interstitial fluid sample.

Said method may be any known method, including, for example, mass spectrometry, immunoassays, chemical assays, liquid chromatography and different indirect and direct photometric methods. For example, some analytical methods may be applied to quantify the marker using mass spectrometry. Generally speaking, the marker is isolated from the biological sample, e.g. by liquid chromatography or two-dimensional gel electrophoresis. The marker is quantified by mass spectrometry, e.g. tandem mass spectrometry associated with liquid chromatography (LC-MS), MALDI-TOF-MS ("matrix-assisted laser desorption/ionization mass spectrometry time-of-flight MS"), etc. The target marker can be quantified by comparison with purified marker standards in known quantities, or by comparison with the quantities of said marker present in the same type of biological samples obtained from healthy controls.

In another embodiment of the invention, the method may be an immunoassay. In general terms, one or more marker ligands are used in the immunoassay. As is used in this invention, a "ligand" is any compound or molecule capable of specifically binding to the target marker. These ligands can be used separately or in combination (antibodies can be used in combination with aptamers, for example).

In an embodiment of the invention, the immunoassay could be a homogenous assay, a heterogeneous assay, an enzyme-immunoassay (EIA, ELISA), a competitive assay, an immunometric assay (sandwich), a turbidimetric assay, a nephelometric assay or other similar assays. Likewise, the immunoassay could be performed manually or with an automatic analyser.

In the method of the invention, said quantity of protein is associated with a disease selected from an inflammatory disease associated to vascular damage, inflammation, a disease associated to abnormal coagulation and cancer. Said disease may also be an autoimmune disease such as lupus, sepsis, shock, preeclampsia, diabetes, unstable angina, in monitoring transplants, restenosis, angioplasty and liver or kidney disease.

According to the method of the invention, the quantity of EPCR protein detected can also be compared with a calibration standard.

An additional object of the present invention is a test kit for the detection and quantification of an EPCR protein which comprises residues 201-256 of the sequence SEQ ID NO:1, e.g. the new EPCR protein of SEQ ID NO: 1, or a fragment of said sequence of residues, preferably of at least 9 amino acids, which comprises:
a) an antibody specific for a polypeptide which comprises residues 201-256 of sequence SEQ ID NO:1, or a fragment of said sequence of residues, or an antibody specific for SEQ ID NO: 1 protein which does not recognise region 1-200 when it is not bound to region 201-256.
b) Reagents to detect a reaction between antibody a) and the EPCR protein which comprises residues 201-256 of sequence SEQ ID NO: 1, or a fragment of said sequence of residues, present in a biological sample.

Preferably, the kit also comprises standards to correlate the quantity of reaction produced with normal and abnormal levels of EPCR protein which comprises residues 201-256 of sequence SEQ ID NO:1.

An additional object of the present invention is the use of a polynucleotide which encodes a polypeptide which comprises residues 201-256 of sequence SEQ ID NO:1, or a fragment of said sequence of residues, for the detection and quantification of a mRNA corresponding to an EPCR protein which comprises residues 201-256 of sequence SEQ ID NO:1, e.g. the new EPCR protein of SEQ ID NO: 1, or a fragment of said sequence of residues, preferably said fragment has at least 9 amino acids. Said EPCR protein is present in a quantity associated to a disease selected from an inflammatory disease associated to vascular damage, inflammation, cancer and a disease associated to abnormal coagulation.

Furthermore, the new soluble form of EPCR of non-proteolytic origin may serve to develop analysis methods which are selective to solely and exclusively detect the presence of this form of soluble EPCR (sEPCRvar), with the advantages this may bring in the detection of specific diseases, without said methods overlapping with those wherein other forms of soluble EPCR or membrane EPCR are detected.

### BRIEF DESCRIPTION OF THE DRAWINGS

**Figure 1**: It shows the different types of EPCR, depending on the way in which the RNA is processed to generate the mature messenger RNA (mRNA) 3 alternative forms of mRNA may be generated, which, in turn, will produce 3 different forms of protein: 1: a mRNA with the 4 exons different from the protein which will be translated as membrane EPCR (mEPCR). 2. A mRNA which only includes exons Ex I, Ex II and Ex III followed by intron three and the entire sequence after this intron, and will be translated as a soluble form of EPCR, disclosed by Esmon in WO-9900673 (altsESPCs). 3. By the new form of alternate cutting and splicing disclosed in the invention, a mRNA will be generated which includes exons Ex I, Ex II and Ex III, but in the processing, exon Ex IV is lost, for which reason, neither the transmembrane domain nor the cytoplasmatic tail are transcribed, and in its place, after Exon III, a sequence which would be a cryptic exon situated in 3' untranslated region appears, in this case the mRNA will be translated as the new soluble EPCR form of the invention (sEPCRvar).
   Soluble EPCR can also be generated by proteolytic activity, possibly from a metalloprotease (still not characterised) which cuts EPCR at membrane height, so that it generates a new soluble form of EPCR (sEPCR), which consists of the fraction of the extracellular receptor and lacks the transmembrane domain and the intracytoplasmatic tail.

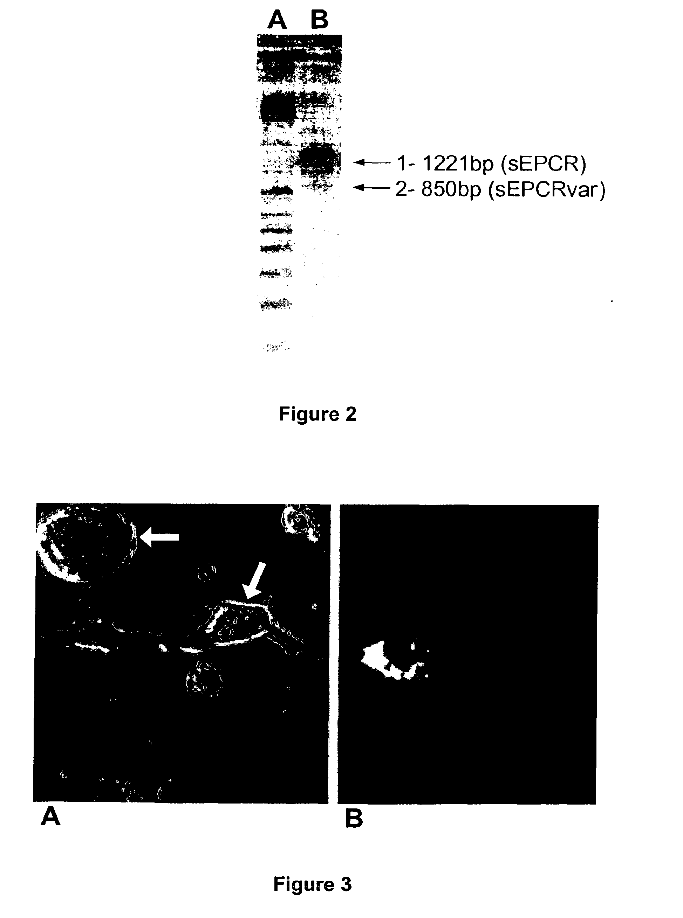

**Figure 2**: Band pattern obtained after the amplification of the HUVEC cDNA with specific EPCR gene primers: in addition to the band corresponding to wild-type EPCR cDNA (1221 bp, arrow 1) a new amplified fragment is observed, corresponding to cDNA of the new variant isoform generated by alternate cut-splice (arrow 2).
**Figure 3**. COS-7 cells transfected with wild-type EPCR (panel A) or with the isoform (panel B), both fused with green fluorescent protein. Panel A shows that wild-type protein tends to accumulate on the cell membrane (arrows), whilst with isoform 3, this localisation pattern is not observed (panel B).

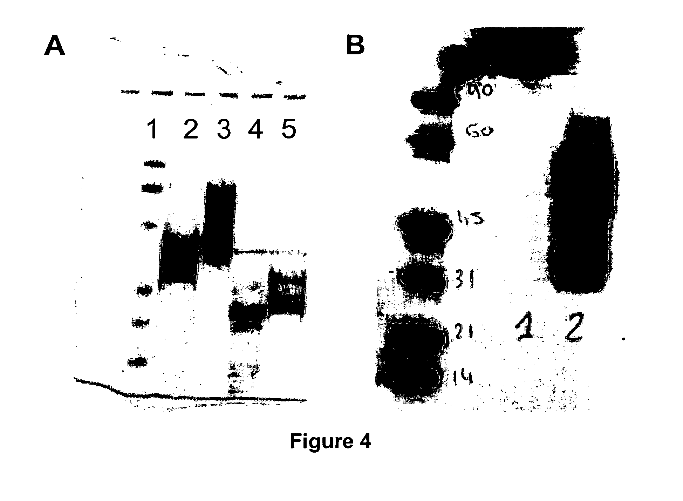

**Figure 4**. Illustrates sEPCRvar expression in *Pichia Pastoris.* The sEPCRvar was purified from the supernatant of stably transformed *P. pastoris* cells, as is indicated in the description. Panel A: Shows different proteins separated by SDS-PAGE and detected using GELCODE Blue dye; Street St: molecular weight marker, indicates the molecular weight of each band expressed in kDa; Street 1: recombinant sEPCR. A diffuse band is observed (due to the intense protein glycosilation) of approximately 42 kDa; Street 2: sEPCRvar. A diffuse band (due to intense protein glycosilation) of 47 kDa is observed. Panel B: Proteins detected by Western blot with the monoclonal anti-EPCR antibody; Street St: molecular weight marker, indicates the molecular weight of each band expressed in kDa; Street 1: blank; Street 2: sEPCRvar. A diffuse band of approximately 47 kDa is observed (that extends between 35 and 60 kDa, due to variable protein glycosilation).

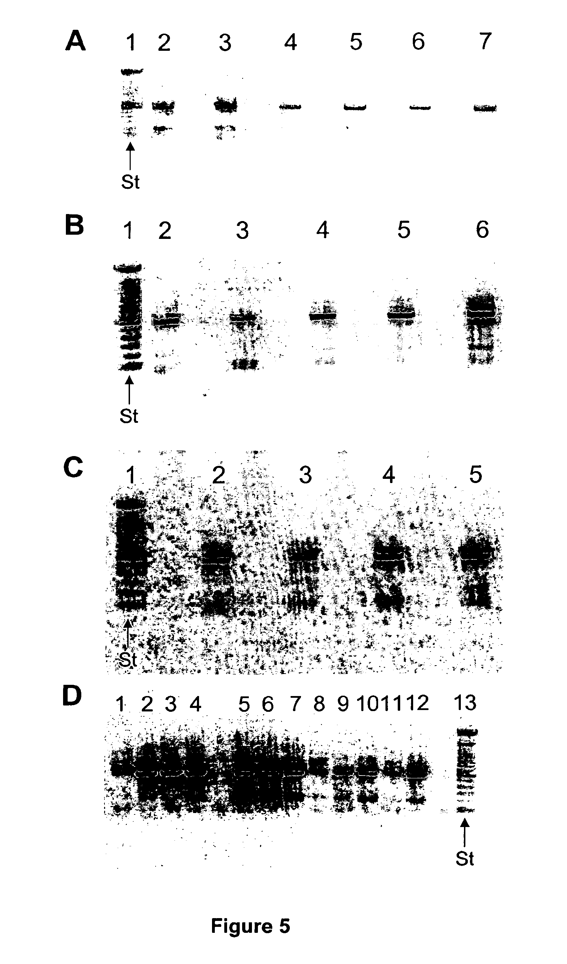

**Figure 5****.** Amplification of EPCR gene from cDNA from different tissues and tumours. Panel A): molecular weight marker, 2: heart, 3: liver, 4: kidneys, 5: pancreas, 6: lung, 7: placenta. Panel B) 1: molecular weight marker, 2: skeletal muscle, 3: brain, 4: thymus, 5: small intestine, 6: spleen. Panel C) 1: marker, 2: prostate; 3: testicle, 4: ovary, 5: colon. Panel D) are samples from tumour lines of lung tumours 2: H 446, 3: H 510, 4: H1264, 5: H 549, 6: H 441, 7: HTB 51, 8: H 676, 9: H 727, 10: H 720, 11: H 385, 12: H 1299.
   The street containing the molecular weight marker is indicated in the figure with the acronym St (from standard). The inferior band of the marker corresponds to a 100bp size; each new band represents a 100bp increment with respect to the previous band (100bp, 200bp, 300bp...).

### EMBODIMENT OF THE INVENTION

### AMPLIFICATION OF EPCR cDNA FROM ENDOTHELIAL CELL cDNA

HUVEC cells were obtained from human umbilical cord vein, cultured according to standard procedures (Jaffe EA, Nachman RL, Becker CG, Minick CR. Culture of human endothelian cells derived from umbilical veins. Identification by morphologic and immunologic criteria. J Clin Inves. 1973 Nov; 52 (11) : 2745-56) and total RNA was extracted following standard techniques, as described in (Perez-Ruiz A, Montes R, Velasco F, Lopez-Pedrera C, Antonio Paramo J, Orbe J, Hermida J, Rocha E. Regulation by nitric oxide of endotoxin-induced tissue factor and plasminogen activator inhibitor-1 in endothelial cells. Thromb Haemost. 2002 Dec; 88 (6) : 1060-5). Resulting RNA is retrotranscribed to cDNA incubating it with transcriptase for 60 minutes at 37°C, followed by five minutes at 65°C for enzyme inactivation. To do this, 120 units (U) of M-MuLV reverse transcriptase (Gibco BRL) were added to 1 µg of RNA in a final volume of 10 µL, which contained 4 µL of the reverse transcriptase buffer solution, 2 mmol/L of deoxynucleotides (dNTPs, Invitrogen), 0.3 µg/mL of random hexamers (Gibco BRL), 0.5 mmol/L of diothiotreitol and 35 U of Rnase inhibitor (RNAsin from human placenta 48,000 U/mL, RNA guard, GE Healthcare Bio-Science).

The expression of the EPCR gene was analysed by polymerase chain reaction (PCR). The primers (Genset, France) used were a 5' primer corresponding to sequence SEQ ID NO: 3 and a 3' primer corresponding to sequence SEQ ID NO:4.

The PCR was performed in a 25 µL volume. Aliquots of cDNA were used, previously obtained by retrotranscription of 1 µg of RNA, to which were added 1 U of taq DNA polymerase (Roche), 2.5 µL of reaction buffer solution (Roche), 0.5 µL of each primer (from a 10 µmol/L solution), 0.25 µL of deoxynucleotides (dNTPs, GE Healthcare Bio-Science) and 0.25 µl of 1 mg/mL BSA. The reaction mixture was adjusted to 25 µL with water. The amplification cycle consists of a 30-second (s) denaturation phase at 94°C, and another annealing and extension phase of 3.5 minutes at 72°C. The cycles are repeated 5 times after which, 32 cycles are performed with the same denaturation phase and another annealing and extension phase of 3 minutes at 68°C. The amplification was terminated maintaining the mixture for 5 minutes at 68°C as a final extension period. The PCR-amplified segment has a length of 1221 base pairs (bp). When the EPCR gene is amplified in these conditions, an additional band appears at a height of approximately 850 bp (Fig 2).

### SEQUENCING OF THE ADDITIONAL BAND OF AMPLIFIED EPCR.

Band 2 was reamplified using the same amplification conditions, but starting from the 850 bp band of Figure 2 as a mould. The amplification product was purified after agarose gel electrophoresis using the commercial kit, QIAquick Gel Extraction Kit, following the manufacturer's instructions. The product thus purified was used as a mould for the sequence reaction (ABI Prism ^{®} BigDyeTM Terminator Cycle Sequencing Ready Reaction Kit). Two sequence reactions were performed, one in the 5'-3' direction and another in the opposite direction using the primers corresponding to sequences SEQ ID NO: 5 and SEQ ID NO: 6.

The result of the reaction was analysed in the ABI Prism 377 DNA Sequencers automatic sequencer. The conclusion is that the new band corresponds to a fragment defined by sequence SEQ ID NO: 7.

The central part of this fragment and whose sequence is SEQ ID NO: 2, corresponds to the cDNA of a new EPCR lacking exon four and part of the 3' untranslated region. The origin of this cDNA is probably an alternate cut-splice phenomenon of the EPCR RNA.

This new species of cDNA encodes a polypeptide whose sequence of amino acids is SEQ ID NO: 1. Residues 1-17 of said sequence correspond to the signal peptide; residues 18-200 correspond to the common extracellular domain of EPCR; residues 201-256 correspond to the new differential domain of this new form of EPCR and replace the transmembrane domain of EPCR. This new domain does not meet the criteria to be a transmembrane domain.

### EXPRESSION OF EPCR AND sEPCRvar FUSED WITH GREEN FLUORESCENT PROTEIN IN MAMMAL CELLS

To study the subcellular localisation of the EPCR isoform, its cDNA was cloned in the pcDNA3.1/CT-GFP-TOPO vector to express it as a fusion protein with green fluorescent protein. The vector thus prepared was used to transiently transform mammal COS-7 cells in culture using Lipofectamine 2000 (Invitrogen); as a control, the same vector was used wherein the cDNA of wild-type EPCR had been cloned, fused with green fluorescent protein. The subcellular localisation of the wild-type EPCR and the isoform were detected by fluorescence microscopy. As is shown in figure 3, wild-type EPCR is mainly localised on the cell membrane, where green fluorescence is observed; nevertheless, the sEPCRvar isoform, in accordance with the inventors' hypothesis, is not localised on the cell membrane.

### CLONING, EXPRESSION, PURIFICATION AND CHARACTERIZATION OF THE EPCR ISOFORM.

### Cloning and expression

To express the identified EPCR variant (EPCRvar), the sEPCR var sequence has been amplified without its signal peptide (residues 17-256) by polymerase chain reaction (PCR) with the initiators
5'-agcttggcatatcgattagccaagacgcctcagatg-3' and
5'-agctatcgtagcggccgcctaccctattatatcagc-3'
which added a ClaI and another NotI restriction sites at 5' and 3' ends respectively, using endothelial cells as cDNA template. These modifications allowed binding the EPCR variant sequence to the ClaI and NotI sites of pPICZαC (Invitrogen) plasmid after the factor α secretion signal of *Saccharomyces cerevisiae,* which permits the efficient secretion of many proteins to the extracellular medium from yeasts. Due to the cloning process, a serine residue and another isoleukin residue were added at the amino end of the sEPCRvar. It was checked that the insert sequence and the vector were checked to be correct using direct sequencing. With the expression vector previously prepared and after linearisation thereof with PmeI restriction enzyme, *Pichia pastoris* cells were transformed using a chemical method (Easy Comp, Invitrogen), giving, as a result, the integration by homologous recombination of the sEPCRvar coding sequence in the methanol response endogenous promoter. The transformation product was cultured in presence of zeocin to select those transformed *P. Pastoris* colonies with the vector that contained the sEPCRvar coding sequence that, in turn, contains the zeocin resistance gene. Briefly, transformed yeasts were cultured in 4 ml of BMY medium [1% (w/v) of yeast extract, 2% (w/v) of peptone, 100 mM potassium phosphate (pH 6,0), 1.34 % (w/v) of yeast nitrogen source with ammonium sulphate, 4x10-5% (w/v) of biotin] supplemented with 1% (v/v) of glycerol (BMGY) and incubated at 28-30°C for approximately 18 hours with stirring. Cells were collected by centrifuging at 2,000g for 5 minutes at room temperature. The supernatant was discarded and sEPCRvar expression was induced with 1% methanol for 18 hours. To do this, cells were resuspended in 3 ml of MBY supplemented with 0.5% (w/v) methanol and were incubated for 18 hours at approximately 28-30°C with vigorous stirring. After the induction, samples from conditioned media were loaded on 12% NuPAGE Bis-Tris gels (Invitrogen, Carlsbad, CA) and the sEPCRvar was detected by Western Blot using RCR-2 monoclonal antibody (Kindly given by Dr. Kenji Fukodome, Saga University, Japan) (Figure 4). For large scale production, the colony which secreted the highest concentration of sEPCRvar was selected. In the colonies selected due to their high sEPCRvar production capacity, their methanol metabolism (quick or slow metaboliser) was studied, which allowed establishing the optimum expression conditions for the most suitable colony. Once the culture conditions and methanol induction were optimised, the scale was augmented to produce large quantities of sEPCRvar.

### Purification

After concentrating and dialysing the *P. Pastoris* culture supernatant against 20 mM Tris-HCl (pH 7.6) without NaCl, two successive purification steps were performed: ion exchange chromatography and gel filtration. In the ion exchange purification, a Resource Q column was used (GE Healthcare Bio-Science) and the elution was performed with a 0.0-300 mM gradient of NaCl in a volume equivalent to 20 columns. The eluted fractions which contained sEPCRvar were united and concentrated by centrifugal ultrafiltration and, then, applied to a Superdex 75-HR10/30 column (GE Healthcare Bio-Science) to perform gel filtration. The concentration of purified protein was determined using BCA total protein assay (Pierre, Rockford, IL) and BSA standards. To detect purified sEPCRvar, samples were loaded on 12% NuPAGE Bis-Tris gels (Invitrogen, Carlsbad, CA) and an electrophoresis was performed in reducing conditions followed by staining with Coomassie blue. An electrophoresis gel was subjected to electroblotting and sEPCRvar was detected with RCR-2 monoclonal antibody. To estimate the molecular weight of the sEPCRvar, a molecular weight standard was included in each gel electrophoresis.

### BIOCHEMICAL ANALYSIS TO CHARACTERISE THE ACTIVITY OF THE NOVEL SOLUBLE EPCR VARIANT

### Affinity of sEPCRvar for PC

Generation of APC in cultured endothelial cells. The cell line used was EA.hy926, a transformed human endothelial cell line which has retained the capacity of expressing thrombomodulin and EPCR (Stearns-Kurosawa DJ, Kurosawa S, Mollica JS, Ferrell GL, Esmon CT. The endothelial cell protein C receptor augments protein C activation by the thrombin-thrombomodulin complex. Proc Natl Acad Sci U S A. 1996; 93: 10212-6). 5x10⁴ cells per well on a 96-well plate were incubated with 0.02 U/ml of thrombin (0.17 nM) (ERL, Swansea, United Kingdom) and increasing concentrations of PC (Baxter, Deerfield, IL. USA) between 50 and 1,000 nM in 20 mM Tris buffer, pH 7.4, supplemented with 150mM NaCl, 5mM CaCl₂, 0.6 mM MgCl₂, 1% BSA, 0.001% Tween-20 and 0.02% NaN₃. After 45 minutes at room temperature, lepirudin (Schering AG, Berlin, Germany) was added at a final concentration of 0.2 µl, to inhibit thrombin and the chromogenic substrate S-2366 (Chromogenix, Milan, Italy) was added 3-4 minutes later at a final concentration of 0.4 mM with the aim of monitoring its proteolysis by APC. The increase in absorbance at 405 nm was kinetically recorded in a microplate reader (iEMS Reader, Labsystems, Finland). The followed by staining with Coomassie blue. An electrophoresis gel was subjected to electroblotting and sEPCRvar was detected with RCR-2 monoclonal antibody. To estimate the molecular weight of the sEPCRvar, a molecular weight standard was included in each gel electrophoresis.

### BIOCHEMICAL ANALYSIS TO CHARACTERISE THE ACTIVITY OF THE NOVEL SOLUBLE EPCR VARIANT

### Affinity of sEPCRvar for PC

Generation of APC in cultured endothelial cells. The cell line used was EA.hy926, a transformed human endothelial cell line which has retained the capacity of expressing thrombomodulin and EPCR (Stearns-Kurosawa DJ, Kurosawa S, Mollica JS, Ferrell GL, Esmon CT. The endothelial cell protein C receptor augments protein C activation by the thrombin-thrombomodulin complex. Proc Natl Acad Sci USA. 1996; 93: 10212-6). 5x10⁴ cells per well on a 96-well plate were incubated with 0.02 U/ml of thrombin (0.17 nM) (ERL, Swansea, United Kingdom) and increasing concentrations of PC (Baxter, Deerfield, IL. USA) between 50 and 1,000 nM in 20 mM Tris buffer, pH 7.4, supplemented with 150mM NaCl, 5mM CaCl₂, 0.6 mM MgCl₂, 1% BSA, 0.001% Tween-20 and 0.02% NaN₃. After 45 minutes at room temperature, lepirudin (Schering AG, Berlin, Germany) was added at a final concentration of 0.2 µl, to inhibit thrombin and the chromogenic substrate S-2366 (Chromogenix, Milan, Italy) was added 3-4 minutes later at a final concentration of 0.4 mM with the aim of monitoring its proteolysis by APC. The increase in absorbance at 405 nm was kinetically recorded in a microplate reader (iEMS Reader, Labsystems, Finland). The adjustment of the curve data to the Michaelis-Menton equation was performed using the Enzfitter program (Biosoft, Cambridge, United Kingdom) which calculated the value of PC activation Km in these conditions.

To study the inhibitory effect of sEPCRvar, 2 µmol/L were added simultaneously with thrombin and PC. Thus, the effect of sEPCRvar on PC activation could be analysed and compared with the effect that sEPCR has on the activation, which reflects its affinity for PC.

Table 1 shows the biochemical characteristics of protein C activation by thrombin on the surface of cultured endothelial cells in the presence or absence of sEPCR and sEPCRvar.

**Table 1**

| | **Km (nM)** | **Vₘₐₓ (AU/ second)** |
|---|---|---|
| PC alone | 49 | 0.0054 |
| PC + 2 µmol/L sEPCR | 881 | 0.008 |
| PC + 2 µmol/L sEPCRvar | 698 | 0.007 |

### The affinity of sEPCR and sEPCRvar for PC

Determination of activated partial thromboplastin time. The activated partial thromboplastin time (APTT) was determined using the Diagnostica Stago Boehringer Mannheim STA Compact equipment (Roche) and Pathromtin reagent (Dade Behring, USA) using a mixture of 5 plasmas from healthy subjects. As is well known, the presence of APC in a mixture of normal plasmas causes the lengthening of APTT. In the experimental conditions used, APTT in absence of APC was 33.1 ± 0.4 s (mean ± SD), whilst when APC was added at a final concentration of 1 nmol/L, APTT was prolonged to 43.9 ± 0.4 s. When sEPCR was added, the effect of APC was reduced so that, in the presence of 1 mmol/L of sEPCR, APTT was 37.2 ± 0.2 s. This inhibitory effect of sEPCR on the anticoagulant effect of APC is dose dependent. The sEPCR added to the mixture of normal plasmas had no direct effect on APTT (33.1 ± 0.4 s in absence compared to 33.9 ± 0.4 s in the presence of sEPCR). All experiments were repeated four times. When, instead of sEPCR, the same concentration of sEPCRvar was added, the result was superimposable to that obtained with sEPCR which demonstrated that sEPCRvar binds APC, as sEPCR does.

Table 2 shows the effect of sEPCR and sEPCRvar on APC anticoagulant function. Coagulation times (APTT) of a mixture of normal plasmas to which 1 nmol/L of APC and different concentrations of sEPCR were added. The mean ± SD of four experiments is represented. The APTT in absence of APC was 33.1 ± 0.4 seconds.

**Table 2**

| **APTT with 1 nmol/L APC (seconds)** | |
|---|---|
| **sEPCR (nmol/L)** | |
| 0 | 43.9 ± 0.4 |
| 10 | 41.8 ± 0.6 |
| 50 | 41.8 ± 0.2 |
| 100 | 40.5 ± 0.6 |
| 250 | 39.9 ± 0.3 |
| 500 | 39.5 ± 0.2 |
| 1000 | 38.2 ± 0.2 |

| **sEPCRvar (nmol/L)** | |
|---|---|
| 0 | 43.9 ± 0.4 |
| 10 | 41.7 ± 0.3 |
| 50 | 41.8 ± 0.9 |
| 100 | 41 ± 0.5 |
| 250 | 39.7 ± 0.2 |
| 500 | 39.4 ± 0.9 |
| 1000 | 37.9 ± 0.3 |

### EXPRESSION ANALYSIS OF sEPCRvar IN DIFFERENT TISSUES AND TUMOUR LINES

To identify the tissues wherein sEPCRvar is expressed, a cDNA library of different human tissues was studied (Multiple tissue cDNA (MTC) panels I and II, Bioscience, USA) by PCR using primers which allow the simultaneous amplification of cDNA corresponding to EPCR and sEPCRvar. The primers (Genset, France) used were:
Primer 5': 5'-GCAGTATGTGCAGAAACATATTTCCGC-3' and
Primer 3': 5'- CATCCCAAGTCTGACACACCTGGAAGT-3'

The PCR was performed in a 25 µL volume. Aliquots of cDNA were used, to which 1 U of taq DNA polymerase (Roche), 2.5 µL of reaction buffer solution (Roche), 0.5 µL of each primer (from a 10 µmol/L solution), 0.25 µL of deoxynucleotides (dNTPs, GE Healthcare Bio-Science) and 0.25 µl of 1 mg/mL BSA were added. The reaction mixture was adjusted to 25 µL with water. The amplification cycle consists of a 30-second (s) denaturation phase at 94°C, and another annealing phase of 1 minute at 56°C and extension of 1.5 minutes at 72°C. The cycles are repeated 35 times. The amplification was terminated maintaining the mixture for 5 minutes at 72°C as a final extension period. The cDNA segment of amplified EPCR had a 578 bp length whilst that of sEPCRvar was 189 bp (Fig. 5). The sEPCRvar form was abundantly expressed in pancreas, heart, liver, kidney, lung, placenta, thymus, small intestine, spleen and colon. The sEPCRvar form was abundantly expressed in some of the tumour lines, especially in H 549, H 441 and H 720. Some of the tumour lines, particularly H 446 and H 676, further expressed another not yet identified form which, in accordance with its electrophoretic mobility, has 1000 bp.

### ANTIBODY PRODUCTION

Immunisation: BALB/C males, of at least 1 month of age, were immunised with sEPCRvar in accordance with the following pattern: three subcutaneous, intradermal or intraperitoneal immunisations or combinations thereof with a quantity of immunogen between 10 and 200 µg in Freund adjuvant or another adjuvant, spaced out at least two weeks; two weeks after the third immunisation, two new booster doses were administered, spaced out 2 days and using the same quantity of immunogen but this time in saline serum. Other animals were also immunised with a mixture of peptides 213-227, 229-242, 212-226, 227-241 and 242-256 of SEQ ID NO:1. These peptides were chosen because they are highly hydrophilic in accordance with the Protscale program, following the algorithm of Kyte J., Doolittle R.F. (J. Mol. Biol., 1982; 157: 105-132).
Fusion. Two days after the last booster dose, hybridomas were produced; a technique which consists of the fusion of animal splenocytes with myeloma cells (SP2/O-Ag14; P3X63-Ag8.6.5.3; P3-NS-1-Ag4-1; etc) in presence of polyethylene glycol 1500 or 4000. This agent enables membrane fusion, so that hybrids will be generated between the various cell types: the hybrid between an animal B lymphocyte and a myeloma cell will form a new cell type, hybridoma, which will produce an antibody and will also be immortal in culture. HAT medium was added to the cell culture to select these hybridomas and eliminate the rest of hybrids: HAT contains aminopterin, inhibitor of *De novo* synthesis pathway of guanosine *(De novo* pathway). Since the myeloma cells lack the functional HPRT enzyme necessary for the nucleic acid synthesis salvage pathway, when the only available pathway, *De novo* pathway, is blocked with aminopterin, they are incapable of synthesising nucleic acids; therefore, after two weeks in culture, the only cells that survive in culture will be splenocyte-myeloma hybrids: splenocytes will give the hybrid guanosine synthesis capacity by the alternative pathway, and myeloma cells the capacity to be indefinitely cultured in in vitro culture.
Detection of anti-sEPCRvar and anti-sEPCR antibody-producing hybridomas. The cells in HAT medium will be distributed in at least ten 96-well culture microplates. Between nine and fourteen days after the fusion, the size of the colonies will be sufficient to analyse the presence of antibodies in the supernatant. To select the hybridomas which secrete antibodies of interest, i.e. antibodies to sEPCRvar, samples of supernatants will be taken from all wells of the five microplates which contain clones, to subject them to an immunoassay: ELISA and Western Blot assays were performed. For the ELISA, plates were coated with sEPCRvar, 0.3 µg/well; after a 15-hour incubation at 4°C and the corresponding blocking with a suitable protein, culture supernatants will be added, corresponding washes performed and then a secondary mouse anti-immunoglobin antibody. In this way, after washing and developing of the wells with peroxidase and o-phenylen diamine, those wells where colour or an increase in absorbance (492 nm) is detected, may contain clones of sEPCRvar antibody secreting hybridomas. For Western Blot, a polyacrylamide gel was run with the recombinant protein and/or a tissue or cell extract, which expresses sEPCRvar protein. It was transferred to a nitrocellulose or PVDF membrane divided in independent zones by a grille-type apparatus to prove the presence of specific antibodies in the supernatants. After blocking and incubating with the specific antibody, it was washed three times and incubated with a secondary antibody conjugated to peroxidase enzyme. The developing was performed using a substrate which on being transformed by the enzyme produces light in the place where it has been bound to the specific antibody. If the luminous band corresponds to a protein with the expected molecular weight, the antibody producing cells from the well from which the supernatant comes, will be grown and frozen in liquid nitrogen.

Since sEPCRvar contains the entire sEPCR portion, the reactivity with the extracellular region of the recombinant EPCR of hybridomas positive for sEPCRvar will be analysed. In this way, those hybridomas that produce antibodies to the specific region of sEPCRvar can be selected.
Monoclonality of the hybridomas. To ensure that each cell culture which secretes an anti-sEPCRvar antibody is monoclonal, cloning or limit dilution techniques are applied: isolated cells will be grown on new culture microplates from the original culture or from stem cells which were positive in the first Western Blot or ELISA test. Once the new colonies arising from one or more cells acquired the sufficient size, supernatants will again be taken from these to subject them to a new ELISA or Western Blot. The colonies which were again positive were subjected to limit dilution and the process repeated until 100% of the analysed supernatants after the third limit dilution contain antibodies to sEPCRvar.
Antibody purification. After culturing the hybridomas in special conditions to obtain a greater yield of antibody concentration (culture bottles with the CELline system from Integra Biosciences or the CELline system from Becton Dickinson), the supernatants containing IgGs will be subjected to purification by liquid chromatography using one or several of the following methodologies: immunoaffinity chromatography, affinity chromatography (in protein A/Protein G/ Protein L, immobilised metal, bioaffinity), cation exchange, hydroxyapatite, hydrophobic interaction, gel filtration, etc. using ÄKTA FPLC equipment, GE Healthcare Bio-Science).

### REFERENCES

1. Bangalore N, Drohan WN, Orthner CL. High affinity binding sites for activated protein C and protein C on cultured human umbilical vein endothelial cells. Independent of protein S and distinct from known ligands. Thromb Haemost 1994; 72: 465-74.
2. Fukodome K, Esmon CT. Identification, cloning and regulation of a novel endothelial cell protein C/activated protein C receptor. J Biol Chem 1994; 269: 26486-91.
3. Stearns-Kurosawa DJ, Kurosawa S, Mollica JS. Ferrell GL, Esmon CT. The endothelial cell protein C receptor augments protein C activation by the thrombin-thrombomodulin complex. Proc. Natl Acad Sci USA 1996; 93: 10212-6.
4. Taylor FB Jr, Peer GT, Lockhart MS, Ferrell G, Esmon CT. Endothelial cell protein C receptor plays an important role in protein C activation in vivo. Blood 2001; 97; 1685-8.
5. Biguzzi E, Merati G, Liaw PC, Bucciarelli P, Oganesyan N, Qu D, Gu JM, Fetiveau R, Esmon CT, msnnucci PM, Faioni EM. A 23bp insertion in the endothelial protein C receptor (EPCR) gene impairs EPCR function. Thromb Haemost 2001; 86: 945-8.
6. Saposnik B, Reny JL, Gaussem P, Emmerich J, Aiach M, Gandrille S: A haplotype of the EPCR gene is associated with increased plasma levels of sEPCR and is a candidate risk factor for thrombosis. Blood 2004: 103; 1311-1318.
7. Uitte de Willige S, Van Marion V, Rosendaal FR, Vos HL, de Visser MCH, Bertina RM. Haplotypes of the EPCR gene, plasma sEPCR levels and the risk of deep venous thrombosis. J. Thromb Haemost. 2004; 2: 1305-10.
8. Taylor FB Jr, Stearns-Kurosawa DJ, Kurosawa S, Ferrell G, Chang AC, Laszik Z, Kosanke S, Peer. G, Esmon CT: The endothelial cell protein C receptor aids in host defense against Escherichia coli sepsis. Blood 2000; 95: 1680-6.
9. Joyce DE, Gelbert L, Ciaccia A, DeHoff B, Grinnell BW. Gene expression profile of antithrombotic protein C defines new mechanisms modulating inflammation and apoptosis. J Biol Chem. 2001; 276: i1199-203.
10. Riewald M, Petrovan RJ, Donner A, Mueller BM, Ruf W. Activation of endothelial cell protease activated receptor 1 by the protein C pathway. Science 2002; 296: 1880-2.
11. Fukudome K, Kurosawa S, Stearns-Kurosawa DJ, He X, Rezaie AR, Esmon CT. The endothelial cell protein C receptor. Cell surface expression and direct ligand binding by the soluble receptor. J Biol Chem 1996; 271: 17491-8.
12. Simmonds, R E, Lane, DA. Structural and functional implications of the intron/exon organisation of the human endothelial cell protein C/activated protein C receptor (EPCR) gene: comparison with the structure of CD1/major histocompatibility complex alpha-1 and alpha-2 domains. Blood 1999; 94: 632-41.
13. Oganesyan V, Oganesyan N, Terzyan S, Qu D, Dauter Z, Esmon NL, Esmon CT. The crystal structure of the endothelial protein C receptor and a bound phospholipid. J Biol Chem 2002; 277: 24851-4.
14. Liaw PC, Mather T, Oganesyan N, Ferrell GL, and Esmon CT. Identification of the protein C/activated protein C binding sites on the endothelial cell protein C receptor. Implications for a novel mode of ligand recognition by a major histocompatibility complex class 1-type receptor. J Biol Chem 2001; 276: 8364-70.
15. Kurosawa S, Stearns-Kurosawa DJ, Hidari N, Esmon CT. Identification of functional endothelial protein C receptor in human plasma. J Clin Invest 1997; 100: 411-8.
16. 8, Xu J, Qu D, Esmon NL, Esmon CT. Metalloproteolytic release of endothelial cell protein C receptor. J Biol Chem 1999; 275: 6038-44.
17. Fukudome K, Esmon CT. Molecular cloning and expression of murine and bovine endothelial cell protein C/activated protein C receptor (EPCR). The structural and functional conservation in human, bovine, and murine EPCR. J Biol Chem. 1995;270:5571-7.
18. Liaw PCY, Neuenschwander PF, Smirnov M, Esmon CT. (2000) Mechanisms by which soluble endothelial cell protein C modulates protein C and activated protein C function. J Biol Chem. 2000; 275:5447-52.
19. Kurosawa S, Esmon CT, Stearns-Kurosawa DJ. The soluble endothelial protein C receptor binds to activated neutrophils: involvement of proteinase-3 and CD11b/CD18. J Immunol 2000; 165: 4697-4703.

## Claims

1. A polypeptide coming from a endothelial protein C receptor, useful as a marker for disease processes in major vessels and cancer, **characterised in that** it comprises residues 201-256 of the sequence SEQ ID NO:1, or a fragment of said sequence of residues between 201 and 256.

2. A polypeptide according to claim 1, **characterised in that** it is a fragment that comprises residues 213-227, 229-242, 212-226, 227-241 242-256 of SEQ ID NO:1.

3. An isolated EPCR protein **characterised in that** it comprises a polypeptide formed from residues 201-256 of sequence SEQ ID NO: 1.

4. An isolated EPCR protein according to claim 3, **characterised in that** it comprises a sequence SEQ ID NO: 1.

5. A recombinant protein **characterised in that** it comprises a polypeptide as defined in any of claims 1 to 4.

6. An isolated or recombinant polynucleotide, **characterised in that** it comprises a sequence which encodes a polypeptide formed from residues 201-256 of sequence SEQ ID NO: 1, or a fragment of said polypeptide.

7. A polynucleotide according to claim 6, **characterised in that** it comes from a cDNA sequence corresponding to SEQ ID NO: 2.

8. A polynucleotide according to one of claims 6 or 7, **characterised in that** it further comprises an operatively bound control sequence that regulates protein or polypeptide expression encoded by said polynucleotide.

9. A DNA construct, **characterised in that** it comprises a sequence which encodes a polypeptide formed by residues 201-256 of sequence SEQ ID NO:1, or a fragment of said polypeptide.

10. A DNA construct according to claim 9, **characterised in that** it comprises a polynucleotide from a cDNA sequence corresponding to SEQ ID NO: 2.

11. A DNA construct according to one of claims 9 or 10, **characterised in that** it further comprises an operatively bound control sequence which regulates the expression of the protein or polypeptide encoded by said polynucleotide.

12. An expression vector, **characterised in that** it comprises a polynucleotide defined in one of claims 6-8, or a DNA construct defined in one of claims 9-11, which encodes a polypeptide which comprises residues 201-256 of sequence SEQ ID NO: 1, or a fragment of said sequence, as defined in claim 1.

13. A host cell, **characterised in that** it comprises a polynucleotide defined in one of claims 6-8, or a DNA construct defined in one of claims 9-11 or an expression vector according to claim 12.

14. A host cell according to claim 13, **characterised in that** the polynucleotide or DNA construct is operatively bound to an appropriate control sequence.

15. A host cell according to one of claims 13 or 14, **characterised in that** said cell is a prokaryotic cell or a eukaryotic cell.

16. A host cell according to claim 15, **characterised in that** said cell is the bacteria *Escherichia coli* or the yeast *Pichia Pastoris.*

17. An expression system **characterised in that** it comprises an expression vector as defined in claim 12 and a host cell as defined in one of claims 13-16.

18. A method to produce a protein, polypeptide or fragment of said polypeptide defined in one of claims 1-5, **characterised in that** it comprises culturing a host cell defined in one of claims 13-16 under conditions which allow the expression of said protein, polypeptide or fragment.

19. A mRNA, **characterised in that** it encodes a protein which comprises residues 201-256 of sequence SEQ ID NO: 1, or fragments of said sequence of residues of at least 9 amino acids.

20. An antibody specific for a polypeptide which comprises residues 201-256 of sequence SEQ ID NO: 1, or a fragment of said sequence of residues which has immunogenic properties.

21. An antibody according to claim 20, **characterised in that** the fragment of the sequence of residues 201-256 of SEQ ID NO:1 has at least 9 amino acids.

22. An antibody according to claim 20, **characterised in that** it is specific for a polypeptide or fragment of said polypeptide whose sequence has been selected from residues 201-256, residues 213-227, 229-242, 212-226, 227-241 242-256, all referred to SEQ ID NO: 1

23. An antibody according to claim 20, **characterised in that** it is specific for SEQ ID NO: 1 and does not recognise region 1-200 when it is not bound to region 201-256.

24. An antibody according to one of claims 20-23, **characterised in that** it is a monoclonal antibody.

25. A method for the *in vitro* selective detection of a biological sample, of an EPCR protein which comprises residues 201-256 of sequence SEQ ID NO: 1, or a fragment of said sequence of residues, as defined in claim 1, **characterised in that** said method comprises:
- obtaining a biological sample,
- analysing the quantity of EPCR protein which comprises residues 201-256 of sequence SEQ ID NO:1, or a fragment of said sequence of residues.

26. A method according to claim 25, **characterised in that** said biological sample is selected from urine, plasma, serum, tissue and interstitial fluid.

27. A method according to one of claims 25-26, **characterised in that** said method is selected from an immunological method, a chromatographic method and a spectrophotometric method.

28. A method according to one of claims 25-27, **characterised in that** said quantity of protein is associated to a disease selected from an inflammatory process associated to vascular damage, inflammation, cancer and a disease associated to abnormal coagulation.

29. A method according to one of claims 25-27, **characterised in that** it comprises comparing the quantity of EPCR protein detected by comparison with a calibration standard.

30. A test kit for the detection and quantification of an EPCR protein which comprises residues 201-256 of sequence SEQ ID NO: 1 or a fragment of said sequence of residues, **characterised in that** it comprises:
a) a specific antibody as defined in one of claims 20 to 24,
b) reagents to detect a reaction between antibody a) and the EPCR protein which comprises residues 201-256 of sequence SEQ ID NO:1, present in a biological sample.

31. A kit according to claim 30, **characterised in that** it also comprises standards to correlate the quantity of reaction produced with normal and abnormal levels of EPCR protein which comprises residues 201-256 of sequence SEQ ID NO: 1.

32. Use of a polynucleotide which encodes a polypeptide which comprises residues 201-256 of sequence SEQ ID NO: 1, for the detection and quantification of a mRNA corresponding to an EPCR protein which comprises residues 201-256 of sequence SEQ ID NO:1, or a fragment of said sequence of residues.

33. Use of a polynucleotide according to claim 32, **characterised in that** said EPCR protein is present in a quantity associated to a disease selected from an inflammatory disease associated to vascular damage, inflammation, cancer and a disease associated to abnormal coagulation.
